(19) Europäisches Patentamt
European Patent Office
Office européen des brevets

(11) Publication number : 0 295 218 B1

# (12) EUROPEAN PATENT SPECIFICATION

(45) Date of publication of patent specification : 22.07.92 Bulletin 92/30

(51) Int. Cl.$^5$ : C07D 239/50

(21) Application number : 88830189.2

(22) Date of filing : 03.05.88

## (54) A process for the preparation of 2,4-diamino-6-(1-piperidinyl)-pyrimidine N-oxide.

(30) Priority : 08.05.87 IT 2043687

(43) Date of publication of application : 14.12.88 Bulletin 88/50

(45) Publication of the grant of the patent : 22.07.92 Bulletin 92/30

(84) Designated Contracting States : AT BE CH DE ES FR GB GR IT LI LU NL SE

(56) References cited : US-A- 3 382 248

(73) Proprietor : ISTITUTO FARMACOLOGICO SERONO SpA
Via Ludovisi 35
I-00187 Roma (IT)

(72) Inventor : Botre', Claudio
Via Emanuele Filiberti, 190
I-00185 Rome (IT)

(74) Representative : Minoja, Fabrizio
Studio Consulenza Brevettuale Via Rossini, 8
I-20122 Milano (IT)

EP 0 295 218 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an improved process for the preparation of 2,4-diamino-6-(1-piperidinyl)-pyrimidine N-oxide of formula (I)

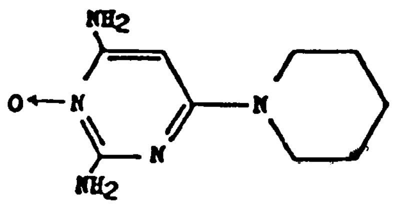

(I)

Compound (I), also known under the common name Minoxidil, has valuable pharmacological activies, particularly as an antihypertonic agent.

A number of preparation processes of compound (I) has been already described, particularly in patent literature; see, for example, USP 3 382 247, USP 3 461 461, USP 3 644 364, DAS 2 114 887, DOS 2 114 887.

According to the literature, compound (I) is obtained starting from 6-chloro-2,4-diamino-pyrimidine of formula (II)

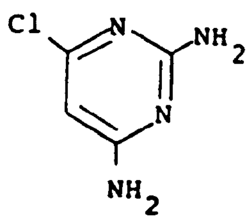

(II)

which is reacted with 2,4-dichlorophenol of formula (III)

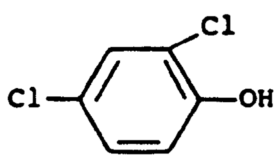

(III)

to obtain 2,4-diamino-6-(2,4-dichloro-phenoxy)-pyrimidine of formula (IV)

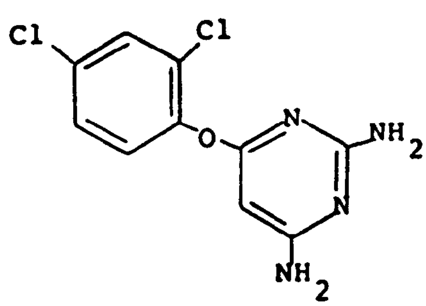

(IV)

which is reacted with 3-chloroperbenzoic acid to give 2,4-diamino-6-(2,4-dichlorophenoxy)-pyrimidine-3-oxide of formula (V)

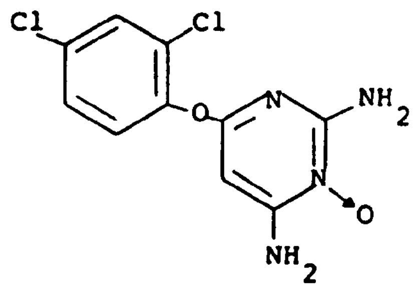

(V)

which on its turn is reacted with piperidine to give compound (I).

The published method involves some drawbacks which make difficult, if not impossible, preparation on industrial scale. Said drawbacks are the following ones:

a) use of 2,4-dichlorophenol, a very caustic and toxic substance, which makes operatively difficult the synthesis step of intermediate IV;

b) during the last reaction step, 2,4-dichlorophenol formes again which, besides having the above cited drawbacks, severely contaminates the final product, which thus becomes difficult to purify;

c) the step number is high, thus involving an increase in labour and material costs.

Now it has been found that 2,4-diamino-6-(1-piperidinyl)-pyrimidine N-oxide can be obtained in higher yields, in a higher purity and with less preparation steps, and therefore more economically, if 6-chloro-2,4-diaminopyrimidine of formula (II)

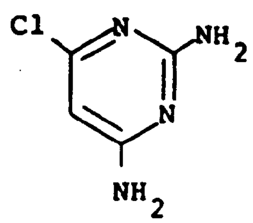

(II)

is reacted with the monoperphthalic acid magnesium salt of formula (VI):

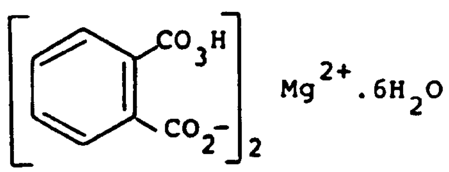

(VI)

to obtain 2,6-diamino-4-chloro-pyrimidine N-oxide of formula (VII):

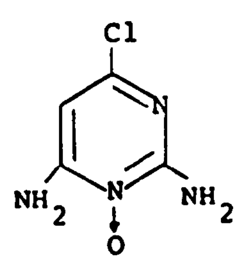

(VII)

This compound directly reacts with piperidine of formula (VIII)

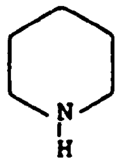

(VIII)

to give compound (I).

The reaction of (II) and (VI) is carried out in a lower alkanol solution, preferably methanol or ethanol, at a temperature from room temperature to the solvent boiling point, preferably at 30-40°C. Compound (III) is obtained very easily, by means of usual techniques, in a purity sufficient to the next step. The second synthetic step is carried out treating intermediate (VII) with piperidine in excess, at the reflux temperature of the reaction mixture, for some hours. At the end of the reaction, the excess reagent is recovered and the product is isolated according to usual techniques.

The starting material for this synthesis, i.e. 6-chloro-2,4-diaminopyrimidine, is easily obtained from ethyl cyanoacetate, which is condensed with guanidine in basic medium, to give 2,6-diamino-4-hydroxy-pyrimidine, which can be transformed into compound (II) by reaction with $POCl_3$.

The following example illustrates the invention in more detail.

## EXAMPLE

a) Preparation of 2,6-diamino-4-hydroxypyrimidine

A 250 l multi-purpose reactor under nitrogen was charged with:

| | |
|---|---|
| ethyl alcohol | 114 l = 89 kg |
| sodium methylate | 23.9 kg |

with stirring. When dissolution was completed, 21.460 kg of guanidine hydrochloride and 25.0 kg of ethyl cyanoacetate were quickly added, after cooling to 20°C.

The mixture was heated to reflux for 2 hours, then concentrated to dryness at room pressure (towards the end of the operation under vacuum). The light brown residue was treated with 80 l of water and left to stand overnight (under nitrogen), then boiled under stirring, distilling the remaining alcohol until the residue temperature reached 90°C. 1.5 kg of active carbon were added, and the reaction mixture was filtered by filter press in another steel reactor. The solution was acidified under stirring with 17 l of conc. hydrochloric acid to pH = 8.0, keeping temperature at 60°C. After cooling, pH was checked and possibly re-adjusted, the mixture was pump filtered, washing with 20 l of water. After drying at 90°C until a water content (K.F.) below 3% was reached, 22.2 kg (yield 79.6%) of a light yellow solid was obtained, unitary by T.L.C. (MeOH4/$CHCl_3$5/(AcOEtl) (Rf 0.3) melting at 285°C.

b) Preparation of 6-chloro-2,4-diaminopyrimidine

In a 200 l multi-purpose glass lined reactor, connected to a sodium hydroxide trap, phosphorous oxychloride (88 l = 147 kg) and 2,6-diamino-4-hydroxypyrimidine (22.2 kg) were charged. The suspension was heated to reflux under stirring for 5 hours, then part of the oxychloride (22 l ) was distilled at room pressure.

The residue was cooled and left to stand overnight, then heated to pre-fluidize the viscous oil, which was charged into a flask provided with a blowdown valve on the bottom. The flask was transferred over a 1000 l glass lined reactor containg 290 l of water. The heated oil was added under stirring, adjusting cooling so that temperature approached as much as possible to 100°C around the end of the addition. At the end of the addition, the solution was refluxed for 10 min., cooled and added with 230 kg of potassium carbonate to a pH from 8.5 to 9.0, under suction. The solid was centrifuged without washing the cake, granulated and dried in oven at 70°C. The dried solid was kneaded at ebollition for 2 hours in 100 l of tetrahydrofuran, the suspension was cooled to room temperature and filtered on a filter press, on which a celite or charcoal layer had been previously placed. The pressed solid was then discharged into the reactor and subjected to the same treatment. The collected filtrates were concentrated to dryness at room pressure and the residual solid was kneaded at ebollition for 30 min. in 25 l of acetone, the suspension was cooled and pump-filtered, and dried first in the air, then in oven at 70°C.

11.9 kg (yield 47.3%) of a light cream solid was obtained, unitary in T.L.C. ($CHCl_3$5/MeOH 4/AcOEt l): Rf 0.6; melting at 197-199°C.

c) Preparation of 2,6-diamino-4-chloro-pyrimidine N-oxide

Methanol (110 l = 87 kg) and 6-chloro-2,4-diaminopyrimidine (11.7 kg) were charged into a 250 l multi-purpose glass lined reactor. The mixture was heated to 30-40°C under stirring until dissolution, then 25 kg of magnesium monoperoxiphthalate was added in 1 hour 30 min. Addition was carried out with cooling, to keep temperature at 30-40°C. When the addition was over, stirring was continued for 5-6 more hours at said temperature, then the reaction mixture was accurately cooled, and pump filtered, washing with some methanol. The thoroughly pressed residue was charged again into the reactor and crystallized from 90 l of water, accurately cooled and pump filtered, washing the cake with water, and dried in oven at 70°C.

8.7 kg (yield 66.9%) of a cream solid was obtained, unitary by T.L.C. ($CHCl_3$ 5/MeOH 4/AcOEt l) Rf 0.4; melting at 189°C (dec).

d) Preparation of 2,4-diamino-6-(1-piperidinyl)-pyrimidine N-oxide

Piperidine (87 l) and 2,6-diamino-4-chloro-pyrimidine N-oxide (8.7 kg) were charged into a 250 l multi-purpose glass lined reactor. The mixture was refluxed for 3 hours under stirring, then concentrated to dryness at room pressure, recovering excess piperidine (operating under vacuum near the end of the operation). Then 22 l of water was added and the mixture was refluxed distilling 3-4 l of the solvent, so to completely remove the remaining piperidine. After throughly cooling, the solid was pump filtered, washing with some water. The day after, the still moist solid was crystallized from demineralized water, filtering at ebollition with 1 kg of charcoal with a filter press, in a glass lined vessel.

The product was pump filtered, washing with some demineralized water. After drying in oven at 80°C, 8.6 kg (yield 75.9%) was obtained of a colorless crystalline product, melting at 260-261°C.

## Claims

1. A process for the preparation of 2,4-diamino-6-(1-piperidinyl)-pyrimidine N-oxide of formula (I):

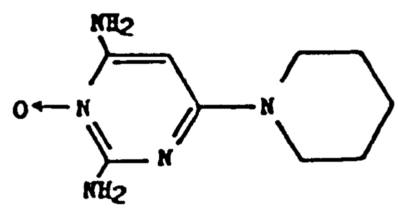

(I)

in which process 6-chloro-2,4-diaminopyrimidine of formula (II)

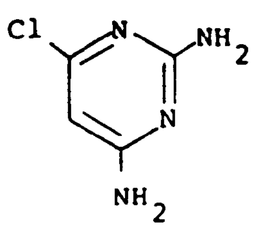

(II)

is reacted with the monoperphthalic acid magnesium salt of formula (VI):

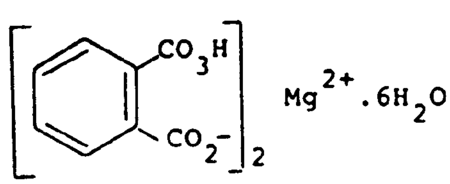

(VI)

to obtain 2,6-diamino-4-chloro-pyrimidine N-oxide of formula (VII):

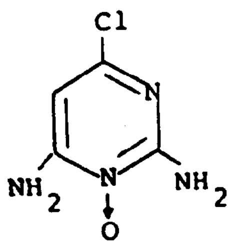

(VII)

which is directly reacted with piperidin (VIII) to give compound (I).

2. A process as claimed in claim 1, in which the reaction of II and VI is carried out in a lower alkanol, at a temperature from 30 to 40°C.

3. A process as claimed in claim 1, in which the reaction of VII and VIII is carried out with an excess of VIII, at the reflux temperature, for some hours.

## Patentansprüche

1. Verfahren zur Herstellung von 2,4-Diamino-6-(1-piperidinyl)-pyrimidin-N-oxid der Formel (I):

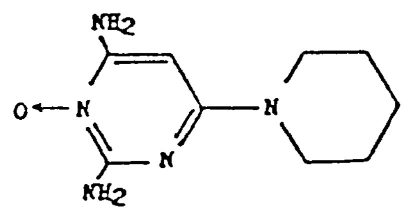

(I)

dadurch gekennzeichnet, daß 6-Chlor-2,4-diaminopyrimidin der Formel (II):

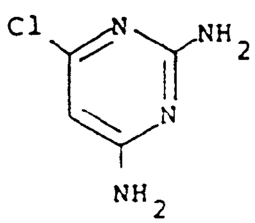

(II)

mit dem Monoperphthalsäure-magnesiumsalz der Formel (VI):

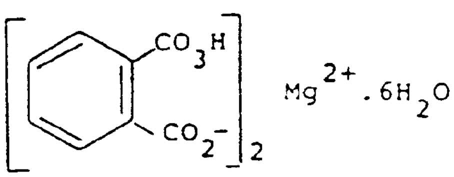

(VI)

unter Bildung von 2,6-Diamino-4-chlorpyrimidin-N-oxid der Formel (VII):

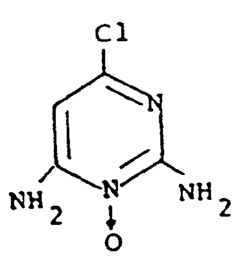

(VII)

umgesetzt wird, welches direkt mit Piperidin (VIII) unter Bildung der Verbindung der Formel (I) umgesetzt wird.

2. Verfahren nach Anspruch 1, dadurch gekennzeichnet, daß die Reaktion von (II) und (VI) in einem niedrigen Alkanol bei einer Temperatur von 30 bis 40°C durchgführt wird.

3. Verfahren nach Anspruch 1, dadurch gekennzeichnet, daß die Reaktion von (VII) und (VIII) mit einem Überschuß an (VIII) bei der Rückflußtemperatur während einiger Stunden durchgeführt wird.

## Revendications

1. Procédé pour la préparation de N-oxyde de 2,4-diamino-6-5 (1-piperidinyl)-pyrimidine de formule (I) :

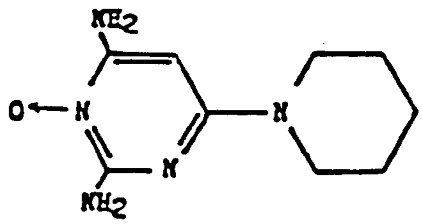

(I)

dans lequel le 6-chloro2,4-diaminopyrimidone de formule (II) :

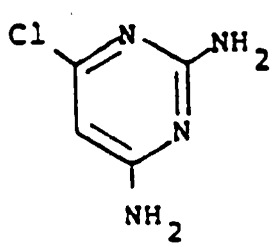

(II)

réagit avec le sel de magnésium de l'acide monoperphtalique de formule (VI) :

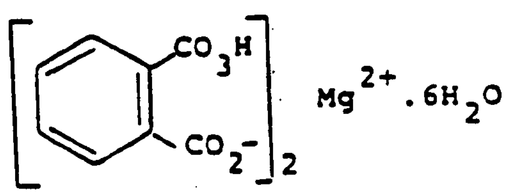

(VI)

pour obtenir le N-oxyde de 2,6-diamino-4-chloropyrimidine de formule (VII) :

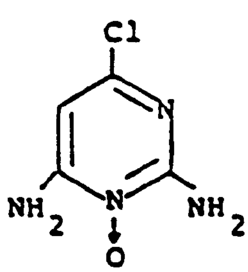

(VII)

qui réagit directement avec la piperidine (VIII), pour donner le composé (I).

2. Procédé selon la revendication 1, dans lequel la réaction entre II et VI est effectuée dans un excès de VIII, à une température entre 30 et 40°C.

3. Procédé selon la revendication 1, dans lequel la réaction de VII avec VIII est effectuée dans un excès de VIII, à la température de reflux, pendant quelques heures.